(19) Europäisches Patentamt
European Patent Office
Office européen des brevets

(11) Veröffentlichungsnummer : **0 196 504**
**B1**

(12) # EUROPÄISCHE PATENTSCHRIFT

(45) Veröffentlichungstag der Patentschrift : **27.07.88**

(51) Int. Cl.⁴ : **C 07 D249/08**

(21) Anmeldenummer : **86103195.3**

(22) Anmeldetag : **10.03.86**

(54) **Verfahren zur Herstellung des (+)-Antipoden des (E)-1-Cyclohexyl-4,4-dimethyl-3-hydroxy-2-(1,2,4-triazol-1-yl)-pent-1-ens.**

(30) Priorität : **19.03.85 DE 3509824**

(43) Veröffentlichungstag der Anmeldung : **08.10.86 Patentblatt 86/41**

(45) Bekanntmachung des Hinweises auf die Patenterteilung : **27.07.88 Patentblatt 88/30**

(84) Benannte Vertragsstaaten : **AT BE CH DE FR GB IT LI NL**

(56) Entgegenhaltungen :
**EP-A- 0 054 431**
**EP-A- 0 114 608**

(73) Patentinhaber : **BAYER AG**
**Konzernverwaltung RP Patentabteilung**
**D-5090 Leverkusen 1 Bayerwerk (DE)**

(72) Erfinder : **Kraatz, Udo, Dr.**
**Andreasstrasse 22a**
**D-5090 Leverkusen (DE)**
Erfinder : **Feyen, Peter, Dr.**
**Mozartstrasse 1**
**D-4020 Mettmann (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein neues Verfahren zur Herstellung des bekannten (+)-Antipoden*) des (E)-1-Cyclohexyl-4,4-dimethyl-3-hydroxy-2-(1,2,4-triazol-1-yl)-pent-1-ens.

Es ist bereits bekannt geworden, den (+)-Antipoden des (E)-1-Cyclohexyl-4,4-dimethyl-3-hydroxy-2-(1,2,4-triazol-1-yl)-pent-1-ens dadurch herzustellen, daß man die entsprechende racemische Verbindung mit einem optisch aktiven Säurechlorid umsetzt, das entstehende Ester-Diastereomeren-Gemisch auf chromatographischem Wege trennt und den Ester, der den (+)-Antipoden enthält, verseift (vgl. DE-OS 3 302 120). Nachteilig an diesem Verfahren ist aber, daß es nur zur Synthese geringer Mengen des gewünschten Antipoden geeignet ist.

Weiterhin ist bereits bekannt geworden, daß man Ketone mit Reduktionsmitteln in Gegenwart verschiedener chiraler Hilfsreagentien zu optisch aktiven Carbinolen reduzieren kann (vgl. Chem. Pharm. Bull. 31, 837 (1983) und EP-OS-O 054 431). Unbefriedigend ist jedoch, daß dieses Verfahren nicht generell anwendbar ist. So lassen sich Ketone, die keine aromatischen Gruppen enthalten, nur in Carbinole mit einer für praktische Zwecke nicht ausreichenden optischen Reinheit überführen.

Es wurde nun gefunden, daß man den (+)-Antipoden des (E)-1-Cyclohexyl-4,4-dimethyl-3-hydroxy-2-(1,2,4-triazol-1-yl)-pent-1-ens der Formel

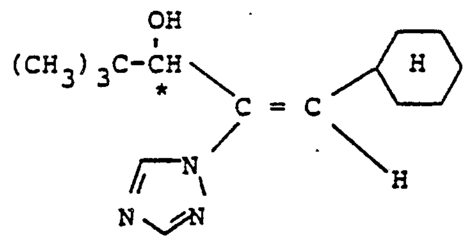

(I)

erhält, indem man (E)-Isomeres des 1-Cyclohexyl-4,4-dimethyl-2-(1,2,4-triazol-1-yl)-pent-1-en-3-ons der Formel

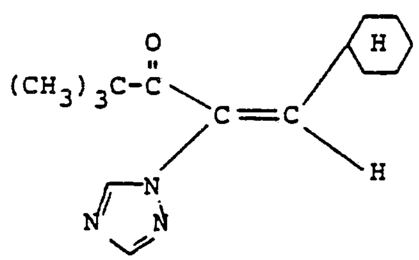

(II)

mit Lithiumaluminium-Hybrid in Gegenwart eines inerten organischen Verdünnungsmittels sowie in Gegenwart eines chiralen Aminoalkohols und gegebenenfalls in Gegenwart eines Amins bei Temperaturen zwischen —80 °C und + 50 °C umsetzt.

Es ist als äußerst überraschend zu bezeichnen, daß sich der (+)-Antipode des (E)-1-Cyclohexyl-4,4-dimethyl-3-hydroxy-2-(1,2,4-triazol-1-yl)-pent-1-ens der Formel (I) nach dem erfindungsgemäßen Verfahren in sehr hoher Ausbeute und ausgezeichneter Reinheit herstellen läßt, denn aufgrund des bekannten Standes der Technik konnte nicht damit gerechnet werden, daß die Umsetzung selektiv zu dem gewünschten Produkt führt.

Das erfindungsgemäße Verfahren zeichnet sich durch eine Reihe von Vorteilen aus. So sind die Reaktionskomponenten auch in größeren Mengen zugänglich und auch im technischen Maßstab problemlos zu handhaben. Ferner ist der zur Durchführung des Verfahrens erforderliche apparative Aufwand gering, und die Aufarbeitung des nach beendeter Umsetzung anfallenden Reaktionsgemisches bereitet keine Schwierigkeiten. Insbesondere jedoch läßt sich der (+)-Antipode des (E)-1-Cyclohexyl-4,4-dimethyl-3-hydroxy-2-(1,2,4-triazol-1-yl)-pent-1-ens der Formel (I) nach dem erfindungsgemäßen Verfahren in höherer Ausbeute und besserer optischer Reinheit herstellen als nach der bisher bekannten Methode, bei der eine klassische Racematspaltung vorgenommen wird.

Der nach dem erfindungsgemäßen Verfahren herstellbare (+)-Antipode des (E)-1-Cyclohexyl-4,4-dimethyl-3-hydroxy-2-(1,2,4-triazol-1-yl)-pent-1-ens ist durch die Formel (I) eindeutig charakterisiert. In dieser Formel ist das asymmetrisch substituierte Kohlenstoffatom, welches das Chiralitätszentrum darstellt, durch ein (*) markiert. Durch den Buchstaben « E » vor dem systematischen Namen der Verbindung der Formel (I) wird ausgedrückt, daß der Cyclohexyl-Rest und der 1,2,4-Triazolyl-Rest auf

*) Unter dem (+)-Antipoden ist hier jeweils dasjenige Enantiomere zu verstehen, das die Schwingungsebene von linear polarisiertem Licht der Natrium-D-Linie nach rechts dreht.

entgegengesetzten Seiten der Doppelbindung stehen.

Verwendet man Lithiumaluminiumhydrid als Reduktionsmittel, (+)-Antipoden des Methylephedrins als chirales Hilfsreagenz, und N-Ethyl-anilin als zusätzliches Amin, so kann der Verlauf des erfindungsgemäßen Verfahrens durch das folgende Formelschema wiedergegeben werden :

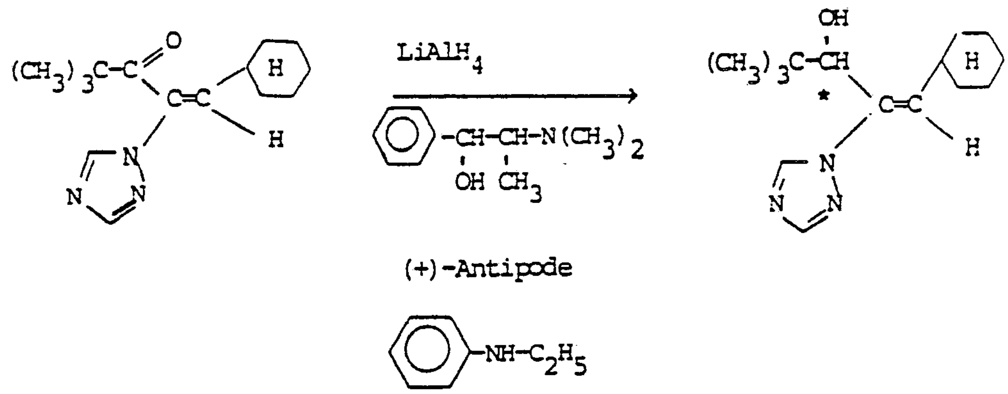


Das bei der Durchführung des erfindungsgemäßen Verfahrens als Ausgangsmaterial benötigte E-Isomere des 1-Cyclohexyl-4,4-dimethyl-2-(1,2,4-triazol-1-yl)-pent-1-en-3-ons der Formel (II) ist bereits bekannt (vgl. DE-OS-3 322 818).

Bei der Durchführung des erfindungsgemäßen Verfahrens fungiert Lithiumaluminium-hybrid als Reduktionsmittel.

Als Verdünnungsmittel können bei der erfindungsgemäßen Umsetzung alle für derartige Reaktionen üblichen inerten organischen Solventien eingesetzt werden. Vorzugsweise in Betracht kommen Ether, wie Diethylether, Tetrahydrofuran und tert.-Butyl-methyl-ether.

Als chirale Hilfsreagentien kommen bei der Durchführung des erfindungsgemäßen Verfahrens optisch aktive Aminoalkohole in Frage. Besonders bevorzugt ist der (+)-Antipode des N-Methyl-ephedrins.

Als Amine, die bei der Durchführung des erfindungsgemäßen Verfahrens gegebenenfalls eingesetzt werden können, kommen vorzugsweise sekundäre Amine in Betracht. Besonders bevorzugt ist N-Ethyl-anilin.

Die Reaktionstemperaturen können bei der Durchführung des erfindungsgemäßen Verfahrens innerhalb eines bestimmten Bereiches variiert werden. Im allgemeinen arbeitet man bei Temperaturen zwischen —80 °C und + 50 °C, vorzugsweise zwischen —70 °C und + 40 °C.

Das erfindungsgemäße Verfahren wird im allgemeinen unter Normaldruck durchgeführt.

Gegebenenfalls arbeitet man unter Schutzgasatmosphäre. Als Schutzgase können dabei alle üblichen unter den Reaktionsbedingungen inerten Gase eingesetzt werden. Vorzugsweise verwendbar sind Stickstoff und Argon.

Bei der Durchführung der erfindungsgemäßen Verfahrens setzt man auf 1 Mol an (E)-Isomerem des 1-Cyclohexyl-4,4-dimethyl-2-(1,2,4-triazol-1-yl)-pent-1-en-3-ons der Formel (II) im allgemeinen 1 bis 5 Mol, vorzugsweise 2 bis 4 Mol, Lithiumaluminium-hybrid und 1 bis 5 Mol, vorzugsweise 2 bis 4 Mol, an chiralem Aminoalkohol sowie gegebenenfalls 2 bis 10 Mol, vorzugsweise 3 bis 8 Mol, an zusätzlichem Amin ein.

Im allgemeinen geht man bei der Durchführung des erfindungsgemäßen Verfahrens so vor, daß man chiralen Aminoalkohol, gegebenenfalls in einem organischen Verdünnungsmittel gelöst, bei Temperaturen zwischen 0 °C und 50 °C, vorzugsweise zwischen 10 °C und 40 °C, in eine Suspension von Lithiumaluminium-hydrid in einem organischen Verdünnungsmittel eintropft, anschließend gegebenenfalls Amin zugibt und danach bei Temperaturen zwischen —80 °C und O °C, vorzugsweise zwischen —70 °C und —10 °C, eine Lösung von (E)-Isomerem des 1-Cyclohexyl-4,4-dimethyl-2-(1,2,4-triazol-1-yl)-pent-1-en-3-ons der Formel (II) in einem organischen Verdünnungsmittels zutropft. Die Aufarbeitung erfolgt nach üblichen Methoden. Im allgemeinen verfährt man in der Weise, daß man Wasser hinzufügt, das Reaktionsgemisch ansäuert, die organische Phase abtrennt, die wäßrige Phase mehrfach mit einem mit Wasser wenig mischbaren organischen Solvens extrahiert, die vereinigten organischen Phasen wäscht und danach einengt. Der verbleibende Rückstand kann durch Digerieren mit geeigneten organischen Solventien oder durch Umkristallisation weiter gereinigt werden.

Der nach dem erfindungsgemäßen Verfahren herstellbare (+)-Antipode des (E)-1-Cyclohexyl-4,4-dimethyl-3-hydroxy-2-(1,2,4-triazol-1-yl)-pent-1-ens der Formel (I) und dessen Verwendung als Fungizid sind bekannt (vgl. DE-OS 3 302 120).

Die Durchführung des erfindungsgemäßen Verfahrens wird durch das folgende Beispiel veranschaulicht.

Herstellungsbeispiel

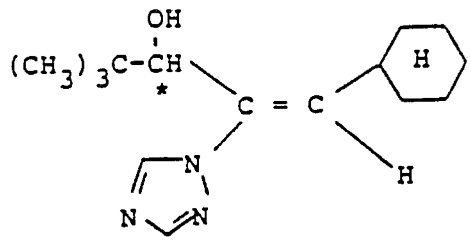

(I)

Eine Lösung von 12,5 g (70 mMol) des (+)-Antipoden des N-Methyl-ephedrins in 140 ml absolutem Diethylether wird bei 20 °C unter Rühren in eine Suspension von 2,7 g (70 mMol) Lithiumaluminiumhydrid in 70 ml Diethylether eingetropft. Man erhitzt 30 Minuten unter Rückfluß, kühlt dann auf 20 °C ab, tropft 17 g (140 mMol) N-Ethylanilin hinzu und läßt eine Stunde unter Rückfluß sieden. Danach wird das Reaktionsgemisch auf —70 °C abgekühlt. Bei dieser Temperatur tropft man eine Lösung von 5,2 g (20 mMol) des (E)-Isomeren des 1-Cyclohexyl-4,4-dimethyl-2-(1,2,4-triazol-1-yl)-pent-1-en-3-ons in 50 ml absolutem Diethylether dazu, Man rührt weitere 4 Stunden bei —70 °C und läßt das Reaktionsgemisch dann noch 16 Stunden bei —70 °C stehen. Anschließend gibt man langsam Wasser hinzu. Das resultierende Reaktionsgemisch wird mit 10 %iger wäßriger Salzsäure angesäuert, so daß der pH-Wert etwa 4 beträgt. Danach wird die organische Phase abgetrennt, und die und die wäßrige Phase wird zweimal mit Essigester extrahiert. Die vereinigten organischen Phasen werden zweimal mit 5 %iger, wäßriger Salzsäure gewaschen und nach dem Trocknen über Magnesiumsulfat unter vermindertem Druck eingeengt. Der verbleibende kristalline Rückstand wird mit Cyclohexan verrührt, abgesaugt, mit Cyclohexan gewaschen und getrocknet. Man erhält auf diese Weise 3,2 g (61 % der Theorie) des (+)-Antipoden des (E)-1-Cyclohexyl-4,4-dimethyl-3-hydroxy-2-(1,2,4-triazol-1-yl)-pent-1-ens in Form eines kristallinen Produktes.

Schmelzpunkt : 164,5 °C

$[\alpha]_D^{20} = + 81{,}4°$ (C = 0,338 mg/10 ml $CHCl_3$)

Das Produkt besitzt eine optische Reinheit von 100 %.

**Patentansprüche**

1. Verfahren zur Herstellung des (+)-Antipoden des (E)-1-Cyclohexyl-4,4-dimethyl-3-hydroxy-2-(1,2,4-triazol-1-yl)-pent-1-ens der Formel

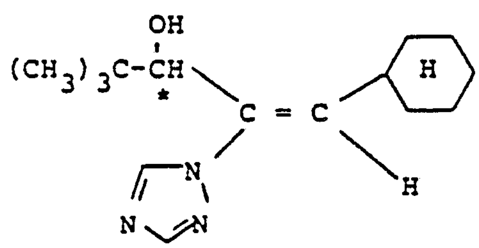

(I)

dadurch gekennzeichnet, daß man (E)-Isomeres des 1-Cyclohexyl-4,4-dimethyl-2-(1,2,4-triazol-1-yl)-pent-1-en-3-ons der Formel

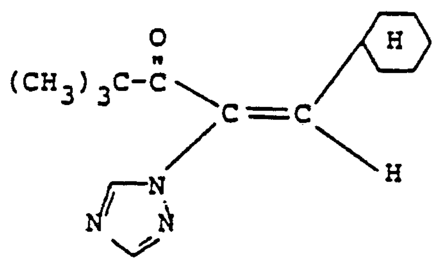

(II)

mit Lithiumaluminium-hybrid in Gegenwart eines inerten organischen Verdünnungsmittels sowie in Gegenwart eines chiralen Aminoalkohols und gegebenenfalls in Gegenwart eines Amins bei Temperaturenzwischen —80 °C und + 50 °C umsetzt.

2. Verfahren gemäß Anspruch 1, dadurch gekennzeichnet, daß man als inerte organische Verdünnungsmittel Diethylether, Tetrahydrofuran oder tert.-Butyl-methyl-ether einsetzt.

3. Verfahren gemäß Anspruch 1, dadurch gekennzeichnet, daß man den (+)-Antipoden des N-Methyl-ephedrins als chiralen Aminoalkohol einsetzt.

4. Verfahren gemäß Anspruch 1, dadurch gekennzeichnet, daß man N-Ethyanilin als Amin einsetzt.

5. Verfahren gemäß Anspruch 1, dadurch gekennzeichnet, daß man die Umsetzung bei Temperaturen zwischen — 70 °C und + 40 °C durchführt.

6. Verfahren gemäß Anspruch 1, dadurch gekennzeichnet, daß man auf 1 Mol en (E)-Isomerem des 1-Cyclohexyl-4,4-dimethyl-2-(1,2,4-triazol-1-yl)-pent-1-en-3-ons der Formel (II) 1 bis 5 Mol Lithiumaluminium-hydrid und 1 bis 5 Mol eines chiralen Aminoalkohols sowie gegebenenfalls 2 bis 10 Mol an Amin einsetzt.

**Claims**

1. Process for the preparation of the (+)-antipode of (E)-1-cyclohexyl-4,4-dimethyl-3-hydroxy-2-(1,2,4-trimazol-1-yl)-pent-1-ene of the formula

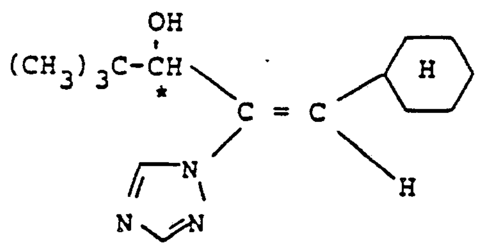

(I)

characterised in that the (E)-isomer of 1-cyclohexyl-4,4-dimethyl-2-(1,2,4-triazol-1-yl)-pent-1-en-3-one of the formula

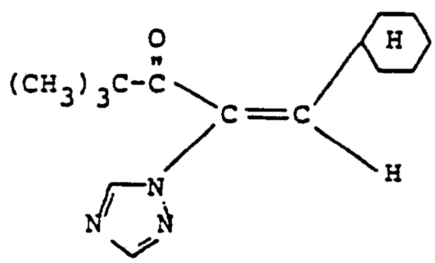

(II)

is reacted with lithium aluminium hydride in the presence of an inert organic diluent and in the presence of a chiral amino alcohol and, if appropriate, in the presence of an amine, at temperatures between — 80 °C and + 50 °C.

2. Process according to Claim 1, characterised in that the inert organic diluent employed is diethyl ether, tetrahydrofuran or tert.-butyl methyl ether.

3. Process according to Claim 1, characterised in that the chiral amino alcohol employed is the (+)-antipode of N-methyl-ephedrin.

4. Process according to Claim 1, characterised in that the amine employed is N-ethylaniline.

5. Process according to Claim 1, characterised in that the reaction is carried out at temperatures between — 70 °C and + 40 °C.

6. Process according to Claim 1, characterised in that 1 to 5 mol of lithium aluminium hydride and 1 to 5 mol of a chiral amino alcohol and, if appropriate, 2 to 10 mol of an amine are employed per mol of the (E)-isomer of 1-cyclohexyl-4,4-dimethyl-2-(1,2,4-triazol-1-yl)-pent-1-en-3-one of the formula (II).

**Revendications**

1. Procédé pour la fabrication de l'antipode (+) du (E)-1-cyclohexyl-4,4-diméthyl-3-hydroxy-2-(1,2,4-triazole-1-yl)-1-pentène de formule

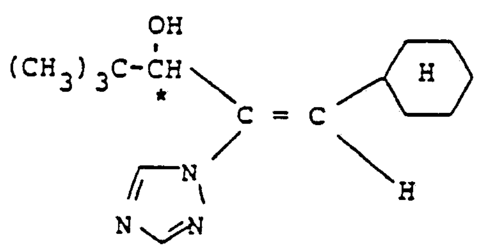

(I)

caractérisé en ce que l'on fait réagir l'isomère (E) de la 1-cyclohexyl-4,4-diméthyl-2-(1,2,4-triazole-1-yl)-1-pentène-3-one de formule

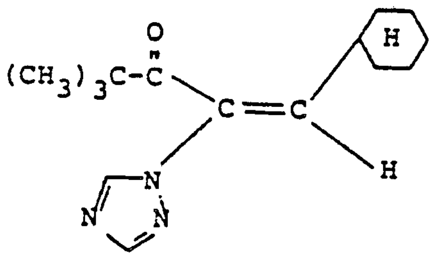

(II)

avec l'hydrure de lithium-aluminium à des températures comprises entre —80 et + 50 °C en présence d'un diluant organique inerte ainsi que d'un aminoalcool chiral et éventuellement en présence d'une amine.

2. Procédé selon la revendication 1, caractérisé en ce que l'on utilise comme diluants organiques l'éther diéthylique, le tétrahydrofuranne ou l'éther tert-butyl-méthylique.

3. Procédé selon la revendication 1, caractérisé en ce que l'on utilise comme aminoalcool chiral l'antipode (+) de la N-méthyl-éphédrine.

4. Procédé selon la revendication 1, caractérisé en ce que l'on utilise comme amine la N-éthylaniline.

5. Procédé selon la revendication 1, caractérisé en ce que l'on met en oeuvre la réaction à des températures comprises entre —70 et + 40 °C.

6. Procédé selon la revendication 1, caractérisé en ce que l'on utilise 1 à 5 mol d'hydrure de lithium-aluminium et 1 à 5 mol d'un aminoalcool chiral ainsi éventuellement que 2 à 10 mol d'amine par mol de l'isomère (E) de la 1-cyclohexyl-4,4-diméthyl-2-(1,2,4-triazole-1-yl)-1-pentène-3-one de formule (II).